# FASCICULE DE BREVET EUROPEEN

(11) **EP 2 091 553 B1**
(45) Date de publication et mention de la délivrance du brevet: **01.08.2012**
(21) Numéro de dépôt: 07857511.5
(22) Date de dépôt: 13.12.2007
(51) Int. Cl.: A61K 38/01, A61K 38/08, C07K 14/47, A23L 1/305, A23L 1/29

(54) **PEPTIDES DERIVES DE LA CASEINE AYANT UNE ACTIVITÉ ANXIOLYTIQUE**
PEPTIDE AUS CASEIN MIT ANXIOLYTISCHER WIRKUNG
CASEIN-DERIVED PEPTIDES HAVING ANXIOLYTIC ACTIVITY

(30) Priorité: 13.12.2006 FR 0610855
(43) Date de publication de la demande: 26.08.2009
(73) Titulaire: Université de Lorraine, 54000 Nancy (FR)
(72) Inventeur: BALANDRAS, Frédérique, 54000 Nancy (FR); MICLO, Laurent, 54600 Villers-Les-Nancy (FR); GAILLARD, Jean-Luc, 14530 Luc Sur Mer (FR); LE ROUX, Yves, 54500 Vandoeuvre-Les-Nancy (FR); LAURENT, François, 54140 Jarville (FR)
(74) Mandataire: Tetaz, Franck Claude Edouard
(86) Numéro de dépôt international: PCT/EP2007/063863
(87) Numéro de publication internationale: WO 2008/071755

(56) Documents cités:
- EP-A1- 0 714 910
- LECOUVEY M ET AL: "TWO-DIMENSIONAL H-NMR AND CD STRUCTURAL ANALYSIS IN A MICELLAR MEDIUM OF A BOVINE ALPHASI-CASEIN FRAGMENT HAVING BENZODIAZEPINE-LIKE PROPERTIES" EUROPEAN JOURNAL OF BIOCHEMISTRY, BERLIN, DE, vol. 248, no. 3, septembre 1997 (1997-09), pages 872-878, XP001037304 ISSN: 0014-2956 cité dans la demande
- GUESDON B. ET AL.: "A tryptic hydrolysate from bovine milk alpha-s1-casein improves sleep in rats subjected to chronic mild stress." PEPTIDES, vol. 27, no. 6, juin 2006 (2006-06), pages 1476-1482, XP005458217
- MICLO L. ET AL.: "Characterization of alph-casozepine, a tryptic peptide from bovine alphas1-casein with benzodiazepine-like activity." FASEB JOURNAL, vol. 15, no. 10, août 2001 (2001-08), pages 1780-1792, XP002481109
- MICHAELIDOU A. ET AL.: "Isolation and identification of some major water-soluble peptides in feta cheese." J. DAIRY SCI., vol. 81, 1998, pages 3109-3116, XP002481110

### SEQUENCE LISTING

<110> Institut National Polytechnique de Lorraine Université Henri Poincaré-Nancy I
<120> Peptides dérivés de la caséine ayant une activité anxiolytique
<130> 362250D26160
<150> FR06/10855
   <151> 2006-12-13
<160> 3
<170> PatentIn version 3.3
<210> 1
   <211> 7
   <212> PRT
   <213> Bos taurus
<400> 1
   Tyr Leu Gly Tyr Leu Glu Gln
   1               5
<210> 2
   <211> 8
   <212> PRT
   <213> Bos taurus
<400> 2
   Tyr Leu Gly Tyr Leu Glu Gln Leu
   1               5
<210> 3
   <211> 9
   <212> PRT
   <213> Bos taurus
<400> 3
   Tyr Leu Gly Tyr Leu Glu Gln Leu Leu
   1               5

## Description

La présente invention concerne des peptides dérivés de la caséine αₛ₁ ayant une activité anxiolytique ainsi que des compositions pharmaceutiques et des compositions alimentaires comprenant ces peptides.

La caséine donne, par diverses techniques de fractionnement, les principales fractions qui sont dénommées respectivement : caséine κ, caséine β, caséine αₛ₁ et caséine αₛ₂. Les séquences en acides aminés de ces caséines sont bien connues, en particulier celle de la caséine αₛ₁ a été déterminée par MERCIER *et al.* (1) et NAGAO *et al.* (2).

Il a été montré que certains fragments peptidiques de ces différentes caséines ont des activités biologiques diverses et notamment des activités opiacées ou anti-opiacées et des activités inhibitrices de l'enzyme de conversion de l'angiotensine 1. Ainsi, les peptides 90-96 et 90-95 de la caséine αₛ₁ ont une nette activité opiacée [ZIOUDROU *et al.* (3) et LOUKAS *et al.* (4)]. Les peptides 23-34 et 194-199, quant à eux, sont des inhibiteurs de l'enzyme de conversion de l'angiotensine 1 [MARUYAMA et SUZUKI (5) et MARUYAMA *et al.* (6)].

Un peptide présentant une activité originale de type anxiolytique a été mis en évidence dans un hydrolysat trypsique de caséine αₛ₁ [EP 0714910 (7)] Il correspond au fragment 91-100 et a été nommé α-casozépine [MICLO *et al.* (8)]. La structure de ce décapeptide a été étudié par ¹H-RMN bidimensionnelle. La séquence comprise entre les résidus de glycine 93 et la leucine 99 adopte, en milieu micellaire, une structure en hélice 3₁₀ initiée et terminée par un tour d'hélice α. Les chaînes latérales des résidus hydrophobes se situent sur la même face de l'hélice alors que les chaînes latérales des résidus hydrophiles se situent sur l'autre face, ce qui confère un caractère amphiphile au peptide et peut lui permettre des interactions avec les membranes. Les interactions ioniques entre le groupement guanidinium du résidu d'arginine 100 et les groupements carboxyliques des résidus d'acide glutamique 96 et d'arginine 100 montrent le rôle capital du résidu d'arginine carboxy-terminal dans la stabilisation de la structure hélicoïdale. Dans une telle structure, les noyaux aromatiques des deux résidus de tyrosine en position 91 et 94 sont orientés de telle façon que la distance entre leur centre (0,56 nm en moyenne) est comparable à la distance observée entre les centres des noyaux aromatiques du nitrazépam, benzodiazépine connue pour ses propriétés anxiolytiques [LECOUVEY *et al.* (9)]. La substitution du résidu d'arginine 100 par un résidu d'alanine diminue fortement l'hélicité du décapeptide et il en résulte une diminution de l'affinité de ce peptide pour le site benzodiazépine du récepteur GABA_{A} d'un facteur 300.000 [Thèse Céline Frochot, (10)]. Ce décapeptide, après absorption orale, peut subir des attaques protéolytiques par les enzymes du tractus digestif ce qui peut diminuer sa biodisponibilité et par conséquent l'empêcher d'atteindre sa cible biologique. Or il est bien connu pour les petits peptides que l'absorption entérocytaire est plus efficace que celle de fragments plus importants et que leur résistance vis-à-vis des protéases digestives est accrue. Ainsi, parmi les fragments générés au cours de la digestion du décapeptide, certains pourraient être plus absorbables et plus résistants, mais au vu des données structurales issues du décapeptide, sans pouvoir conserver la moindre activité de type anxiolytique.

De manière surprenante, des peptides dérivés du décapeptide et ne contenant pas le résidu-clé d'arginine 100 montrent dans des tests comportementaux *in vivo* chez le rat des propriétés anxiolytiques. Au vu de leur petite taille, ces peptides sont plus facilement absorbables et moins facilement dégradables.

### Listage des séquences

SEQ ID NO. 1: Heptapeptide correspondant aux positions 91-97 de la caséine αₛ₁
SEQ ID NO. 2 : Octapeptide correspondant aux positions 91-98 de la caséine αₛ₁
SEQ ID NO. 3 : Nonapaptide correspondant aux positions 91-99 de la caséine αₛ₁

### Description de l'invention

La présente invention a pour objet des peptides isolés ayant l'une des SEQ ID NOs. 1 ou 3.

L'invention a aussi pour objet des polynucléotides codant pour les peptides des SEQ ID NOs. 1 ou 3.

Un autre objet de l'invention est un organisme hôte à l'exclusion de l'homme exprimant un peptide selon l'une des SEQ ID NOs. 1 ou 3.

Enfin, l'invention concerne aussi un hydrolysat enzymatique de la caséine comprenait un peptide selon l'invention.

Dans un autre aspect, l'invention se rapporte aux peptides selon les SEQ ID NOs. 1-3 et aux hydrolysats enzymatiques de la caséine selon l'invention pour la thérapie. De préférence, pour le traitement de l'anxiété, des troubles du sommeil et de l'épilepsie.

L'invention se rapporte aussi à des compositions pharmaceutiques contenant à titre de principe actif une quantité efficace d'un peptide selon l'invention et/ou une quantité efficace d'un hydrolysat selon l'invention en combinaison avec un véhicule pharmaceutique approprié.

L'invention concerne également l'utilisation d'un peptide selon l'invention et/ou d'un hydrolysat selon l'invention pour l'obtention d'un médicament pour le traitement de l'anxiété, des troubles du sommeil et de l'épilepsie.

Un autre objet de la présente invention est une composition alimentaire contenant une quantité efficace d'un peptide selon l'invention et/ou une quantité efficace d'un hydrolysat selon l'invention.

L'invention se rapporte aussi à un complément alimentaire contenant une quantité efficace d'un peptide selon l'invention et/ou une quantité efficace d'un hydrolysat selon l'invention en combinaison avec des supports alimentaires de nature protéique ou glucidique.

Un autre objet de la présente invention est un procédé de préparation de peptides ayant une activité anxiolytique caractérisé en ce qu'il comprend les étapes suivantes :
- hydrolyse enzymatique de la caséine ;
- isolement d'au moins un peptide choisi parmi les peptides ayant les SEQ ID NOs. 1 ou 3.

Ainsi la présente invention a pour objet des peptides dérivés de la caséine αₛ₁ dont la séquence est représentée aux SEQ ID NOs. 1 ou 3 sont décrits des peptides dont la séquence en acides aminés est choisie parmi : Tyr-Leu-Gly-Tyr-Leu-Glu-Gln, Tyr-Leu-Gly-Tyr-Leu-Glu-Gln-Leu et Tyr-Leu-Gly-Tyr-Leu-Glu-Gln-Leu-Leu. Dans un mode de réalisation préféré, l'invention se rapporte à l'heptapeptide de la SEQ ID NO. 1 ayant la séquence en acides aminés Tyr-Leu-Gly-Tyr-Leu-Glu-Gln.

Dans un deuxième aspect, l'invention se rapporte à des polynucléotides codant pour les peptides selon l'invention. En raison de la dégénérescence du code génétique, différents polynucléotides peuvent coder pour un même peptide. Selon la présente invention, on entend par "polynucléotide " une chaine nucléotidique simple brin ou son complémentaire pouvant être de type ADN ou ARN, ou une chaine nucléotidique double brin pouvant être de type ADNc (complémentaire) ou génomique. De préférence, les polynucléotides de l'invention sont de type ADN, notamment d'ADN double brin. Le terme "polynucléotide" désigne également les polynucléotides modifiés. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

Un autre objet de l'invention est un organisme hôte exprimant un peptide selon l'une des SEQ ID NOs. 1 ou 3. Les peptides de la présente invention peuvent être exprimés et produits dans différents organismes hôtes selon des techniques bien connues de l'homme du métier. Typiquement, l'organisme hôte est transformé avec une cassette d'expression comprenant un polynucléotide codant pour un peptide selon l'invention. Ce polynucléotide peut être intégré dans le génome de l'organisme hôte ou se répliquer de manière stable dans l'organisme hôte. Par organisme hôte, on entend en particulier selon l'invention tout organisme mono ou pluricellulaire, inférieur ou supérieur, en particulier choisi parmi les bactéries, les levures, les champignons et les mammifères. Par organisme hôte, on entend un organisme non humain. Les peptides de l'invention peuvent donc être produits puis isolés ou purifiés à partir d'organismes hôtes transformés les exprimant.

De préférence, les peptides selon l'invention sont obtenus à partir de la caséine du lait et plus préférentiellement à partir de la caséine αₛ₁. La caséine utilisée est de préférence la caséine du lait de bovins et plus préférentiellement de vaches laitières (*Bos taurus*). La séquence de la caséine αₛ₁ est référencée dans la base de données Swiss Prot sous le numéro P02662. Après fractionnement des protéines du lait, la caséine est « digérée » ou hydrolysée avec des enzymes appropriées pour obtenir les peptides selon l'invention. Dans un premier mode de réalisation, la caséine du lait est directement hydrolysée avec des enzymes pour obtenir les peptides souhaités. Dans ce mode de réalisation, il peut être nécessaire de purifier partiellement ou totalement les peptides selon l'invention après l'hydrolyse. Dans un deuxième mode de réalisation selon l'invention, l'hydrolyse enzymatique s'effectue directement sur de la caséine αₛ₁. Dans ce mode de réalisation, l'hydrolysat obtenu sera déjà enrichi en peptides selon l'invention et des étapes de purification supplémentaires ne sont souvent pas nécessaires. L'homme du métier choisira les enzymes appropriées pour obtenir les peptides souhaités. Ces techniques sont bien connues de l'homme du métier et décrites dans la littérature. L'invention a donc également pour objet un hydrolysat de la caséine comprenant un peptide selon l'invention. De préférence, il s'agit d'un hydrolysat de la caséine αₛ₁.

Les peptides de la présente invention ont un effet anxiolytique. L'invention concerne l'utilisation de ces peptides notamment pour le traitement de l'anxiété, des troubles du sommeil, de l'épilepsie.

L'invention se rapporte donc également aux peptides selon les SEQ ID NOs. 1-3 et aux hydrolysats enzymatiques de la caséine αₛ₁ selon l'invention pour le traitement de l'anxiété, des troubles du sommeil et de l'épilepsie.

L'invention se rapporte aussi à des compositions pharmaceutiques contenant à titre de principe actif une quantité efficace d'un peptide selon l'invention et/ou une quantité efficace d'un hydrolysat selon l'invention en combinaison avec un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Les peptides et hydrolysats selon l'invention peuvent être employés en thérapie seuls, ou en combinaison avec au moins un autre agent actif. Ces autres agents actifs sont en particulier choisis parmi les actifs appropriés pour le traitement de l'anxiété, des troubles du sommeil et de l'épilepsie. Il peut s'agir d'adjuvants permettant d'améliorer l'activité des composés selon l'invention, ou encore d'autres actifs connus pour leur emploi dans le traitement des dites affections. De tels agents actifs sont bien connus de l'homme du métier, disponibles dans le commerce ou encore décrits dans des ouvrages de référence comme Le Dictionnaire Vidal, édité avec mises à jours chaque année.

La présente invention concerne donc également un produit comprenant un composé selon l'invention et un autre agent actif comme produit de combinaison pour une utilisation simultanée, séparée ou étalée dans le temps en thérapie, et en particulier dans le traitement de l'anxiété et des troubles du sommeil. Ces autres agents actifs sont en particulier choisis parmi les actifs appropriés pour le traitement de l'anxiété comme par exemple les composés de la classe des benzodiazépines ou les inhibiteurs de la recapture de la sérotonine.

L'invention concerne également l'utilisation d'un peptide selon l'invention et/ou d'un hydrolysat selon l'invention pour l'obtention d'un médicament pour le traitement de l'anxiété, des troubles du sommeil et de l'épilepsie.

Un autre objet de la présente invention est une composition alimentaire contenant une quantité efficace d'un peptide selon l'invention et/ou une quantité efficace d'un hydrolysat selon l'invention. Par composition alimentaire, on entend tout ce qui est propre à servir d'aliment. Les peptides peuvent être utilisés comme principe actif soit dans des compositions alimentaires en combinaison avec des supports alimentaires de nature protéique ou glucidique, soit dans des produits alimentaires destinés à une alimentation particulière. Les peptides ou les hydrolysats selon l'invention peuvent également être administrés sous forme de compléments alimentaires. Ces compléments alimentaires conviennent pour supplémenter l'alimentation des personnes sujettes notamment à l'anxiété, aux troubles du sommeil et à l'épilepsie.

L'invention concerne également des procédés d'obtention des peptides et des hydrolysats selon l'invention. La caséine entière est obtenue à partir du lait par précipitation acide et neutralisation à l'aide d'un alcali selon des procédés bien connus. Par exemple, on peut utiliser la méthode de NITSCHMANN et LEHMANN (11). La caséine ou la caséine αₛ₁, utilisées comme produit de départ pour l'obtention des peptides selon l'invention, peuvent être obtenues par les procédés classiques bien connus de l'homme du métier à partir du lait, de caséines entières, de caséinates et de concentrés de protéines totales du lait, obtenus par exemple selon les procédés décrits par THOMSON (12) et MAUBOIS (13). Par exemple, on peut préparer la caséine αₛ₁ en mettant en oeuvre la méthode décrite par SANOGO *et al.* (14). Cette méthode est une méthode de fractionnement sur DEAE-cellulose utilisant un gradient discontinu de chlorure de calcium comme éluant. Elle présente l'avantage de séparer rapidement l'ensemble des caséines. Elle peut être avantageusement mise en oeuvre avec, comme support échangeur d'anions, la DEAE-cellulose DE 52 [commercialisée par WHATMAN *Ltd*, Springfeld, Grande-Bretagne] qui est une résine pré-conditionnée ne nécessitant aucun pré-cycle acido-basique avant sa première utilisation. Les peptides peuvent ensuite être obtenus par hydrolyse de la caséine avec des enzymes appropriées. Les peptides peuvent ensuite être concentrés ou isolés par chromatographie liquide haute performance (CLHP) en phase inverse, par chromatographie liquide haute performance d'échange d'anions ou par chromatographie de gel filtration de seuil 1.000 Da ou par centrifugation sur membrane et autres techniques de séparation sur membrane (microfiltration, ultrafiltration, etc.).

Un autre objet de la présente invention est donc un procédé de préparation de peptides ayant une activité anxiolytique caractérisé en ce qu'il comprend les étapes suivantes :
- hydrolyse enzymatique de la caséine ;
- isolement d'au moins un peptide choisi parmi les peptides ayant les SEQ ID NOs. 1 ou 3.

Par isolement, on entend la purification partielle ou totale des peptides selon l'invention ou simplement l'enrichissement de l'hydrolysat obtenu en peptides selon l'invention. Cet enrichissement peut s'effectuer par exemple par fractionnement de l'hydrolysat obtenu. Alternativement, l'hydrolysat obtenu à partir de caséine αₛ₁ contenant au moins un peptide selon l'invention peut être utilisé directement pour l'obtention des compositions pharmaceutiques et alimentaires selon l'invention.

Les peptides peuvent aussi être obtenus par synthèse peptidique selon les procédés bien connus de l'homme de l'art, tels que ceux décrits par exemple par MERIFFIELD (15).

Les hydrolysats, les compositions pharmaceutiques et alimentaires selon l'invention peuvent contenir un ou plusieurs peptides selon l'invention. Dans un mode de réalisation préféré, l'invention concerne des hydrolysats, des compositions pharmaceutiques et alimentaires contenant l'heptapeptide de la SEQ ID NO. 1.

L'invention va être maintenant décrite plus en détail par les exemples ci-après non limitatifs.

### Figures

| | |
|---|---|
| Figure 1 : | Test d'enfouissement défensif conditionné |
| Figure 2 : | Test du labyrinthe en croix surélevé |
| Figure 3 : | Test de la boîte claire/obscure |

### Exemples

### 1. Modalités d'obtention de l'heptapeptide

La caséine entière est obtenue à partir du lait par précipitation acide et neutralisation à l'aide d'un alcali selon des procédés bien connus.

L'heptapeptide ayant la séquence en acides aminés ci-après : Tyr-Leu-Gly-Tyr-Leu-Glu-Gln de masse moléculaire de 884 Da, correspond au peptide 91-97 de la caséine aₛ₁. Il peut être obtenu à partir de la caséine αₛ₁ par hydrolyse enzymatique, en particulier à l'aide de la trypsine et ensuite de la pepsine. L'hydrolyse par la trypsine de la caséine αₛ₁ libère des fragments peptidiques divers. L'ensemble des fragments trypsiques subissent une hydrolyse par la pepsine A (EC 3.4.23.1) (issu de muqueuse gastrique porcine, avec une activité de 3.200-4.500 unités/mg de protéine), dans un rapport E/S=1/200 à un pH acide, dans un tampon et à une température optimum pour l'activité de l'enzyme pendant 240 minutes. Cette dernière hydrolyse libère de nouveaux fragments dont l'heptapeptide 91-97 de la caséine αₛ₁. L'heptapeptide peut être ensuite concentré ou isolé par chromatographie liquide haute performance (CLHP) en phase inverse, par chromatographie liquide haute performance d'échange d'anions ou par chromatographie de gel filtration de seuil 1.000 Da ou par centrifugation sur membrane et autres techniques de séparation sur membrane (microfiltration, ultrafiltration, etc.).

### 2. Etudes pharmaco-comportementales chez le rat Wistar

### 2.1. Enfouissement défensif conditionné

### 2.1.1. Principe

Le test de l'enfouissement défensif conditionné a été utilisé pour mesurer le niveau d'anxiété chez le rat. Développé par PINEL et TREIT (16), ce test est basé sur la tendance innée des rongeurs, placés dans un environnement familier, à enfouir tout objet perçu comme dangereux en projetant sur celui-ci la litière présente au niveau du sol. L'expression du comportement d'enfouissement par l'animal est représentatif de son niveau d'anxiété : plus il est anxieux, plus il enfouit. Ainsi, une électrode délivrant un choc unique de faible intensité provoque l'apparition du comportement d'enfouissement chez le rat et l'administration de molécules à propriétés anxiolytiques réduit significativement la durée d'expression de ce comportement.

### 2.1.2. Appareillage

Le test se déroule dans une boîte en Plexiglas (44 x 28 x 18 cm) dont l'une des parois présente un orifice permettant d'insérer une électrode (7 x 2 x 0,5 cm). Le fond de la boîte est recouvert d'une épaisse couche de sciure (5 cm). L'électrode est reliée à un générateur délivrant un courant d'une intensité de 1 mA lorsqu'il est activé par l'expérimentateur. L'ensemble de l'expérimentation est réalisé en lumière de faible intensité.

### 2.1.3. Protocole

L'expérimentation se compose d'une phase d'habituation de 2 jours suivie d'une phase de test. Lors de la phase d'habituation, les rats sont placés 20 min par jour dans la boîte de test, sans l'électrode, de manière à les habituer au dispositif expérimental. Lors de la phase de test, chaque rat est placé individuellement dans la boîte qui contient l'électrode. Lorsque l'animal touche l'électrode pour la première fois, il reçoit un unique choc. A partir de cet instant, son comportement est filmé pendant 5 min et les variables comportementales quantifiées.

### 2.1.4. Observations

La durée totale d'enfouissement et la latence d'apparition du comportement d'enfouissement après le choc sont les variables les plus importantes. Est également comptabilisé un certain nombre de comportements orientés vers l'électrode et corrélés avec le niveau d'anxiété du rat (approches, fuites, contacts). Enfin, des indicateurs du niveau d'activité du rat tels que le nombre de redressements sont relevés.

### 3.1.5. Traitements

L'étude a portée sur 23 rats répartis en trois groupes :
- un groupe témoin (8 rats) ayant reçu 1 mL/kg de sérum physiologique [NaCl 0,9% (m/v)],
- un groupe traité avec du diazépam (Valium®, Roche, Neuilly-sur-Seine, France) à la dose de 0,5 mg/kg (8 rats) servant de contrôle positif,
- un groupe traité avec le fragment 91-97 de la caséine αₛ₁, préalablement dissous dans une solution de NaCl 0,9% (m/v), à la dose de 0,5 mg/kg (7 rats).

Les animaux reçoivent leur traitement respectif par voie intra-péritonéale une demi-heure avant le passage dans le test d'enfouissement défensif conditionné.

### 2.1.6. Résultats

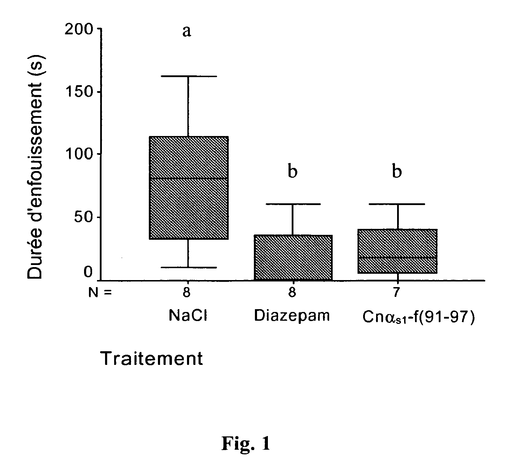

L'effet de l'injection intrapéritonéale chez le rat Wistar de 1 mL/kg de NaCl 0,9 % (m/v) (n=8), ou de 0,5 mg/kg de diazépam (n=8) ou de 0,5 mg/kg de l'heptapeptide CNαₛ₁-(f91-97) (n=7) sur la durée d'enfouissement d'une sonde aversive après un choc unique de 1 mA a été étudiée par analyse de variance non paramétrique (Kruskall-Wallis) et les comparaisons de moyennes ont été réalisées par le test de Mann-Whitney (a,b moyennes pour chaque traitement significativement différentes *p* < 0,05, figure 1). La durée d'enfouissement des animaux traités avec le NaCl 0,9% (m/v) est significativement supérieure à celle des animaux traités par le diazépam (*p* < 0,02) et à celle des animaux traités par le fragment 91-97 de la caséine αₛᵢ (*p* < 0,04). Aucune différence significative n'est constatée entre le diazépam et le fragment 91-97 de la caséine αₛ₁. En conséquence, le fragment 91-97 de la caséine αₛ₁ présente une action anxiolytique chez le rat Wistar qui va dans le même sens que celle du diazépam.

### 2.2. Labyrinthe en croix surélevé

### 2.2.1. Principe

Le test du labyrinthe en croix surélevé permet de mesurer la densité du comportement exploratoire dans une situation nouvelle aversive [PELLOW *et al.,* 1985 (17)]. L'expérience exploite le conflit, chez les rongeurs, entre la peur des espaces ouverts et le désir d'explorer un nouvel environnement. Le rat, placé au centre du dispositif, est contraint d'explorer son environnement. Les bras ouverts constituent un environnement anxiogène, alors que ce n'est pas le cas des bras fermés. Le nombre d'entrées dans l'ensemble des bras, celui concernant les bras ouverts, les bras fermés, le temps passé dans les bras ouverts, la latence d'entrée dans le premier bras ouvert constituent des paramètres reflétant le degré d'anxiété de l'animal.

### 2.2.2. Appareillage

Le labyrinthe en croix surélevé, construit en bois, est constitué de quatre branches à 90° les unes des autres. Les deux branches ouvertes (45 x 10 cm) et les deux branches fermées (45 x 10 cm ; entourées de plaques de bois sur une hauteur de 40 cm) sont disposées en vis-à-vis. Le carré central mesure 10 x 10 cm. Les branches ouvertes sont éclairées par une lumière blanche d'une intensité de 500 lux. Le labyrinthe en croix surélevé se situe à 50 cm du sol.

### 3.2.3. Protocole

Les observations ont lieu entre 9 h et 11h du matin pour minimiser l'influence du rythme circadien. Avant chaque passage dans le labyrinthe en croix surélevé, l'animal est placé pendant 10 min dans un openfield afin de stimuler son comportement exploratoire. Il est ensuite placé dans le carré central du labyrinthe en croix surélevé et observé pendant 5 min. Le labyrinthe en croix est lavé avec de l'éthanol à 95% (v/v) entre deux passages afin d'éliminer les odeurs.

### 2.2.4. Traitements

L'étude a portée sur 45 rats répartis en trois groupes :
- un groupe témoin (15 rats) ayant reçu 2 mL/kg d'une solution contenant 1% (v/v) de glycérol et 0,2% (v/v) de méthylcellulose (véhicule),
- un groupe traité avec du diazépam (Valium^{®}, Roche, Neuilly-sur-Seine, France) à la dose de 1 mg/kg mis en suspension dans le véhicule (15 rats) servant de contrôle positif,
- un groupe traité avec le fragment 91-97 de la caséine αₛ₁, préalablement dissous dans le véhicule, à la dose de 0,7 mg/kg (15 rats).

Les animaux reçoivent leur traitement respectif par voie intra-péritonéale une demi-heure avant le passage dans l'openfield.

### 2.2.5. Résultats

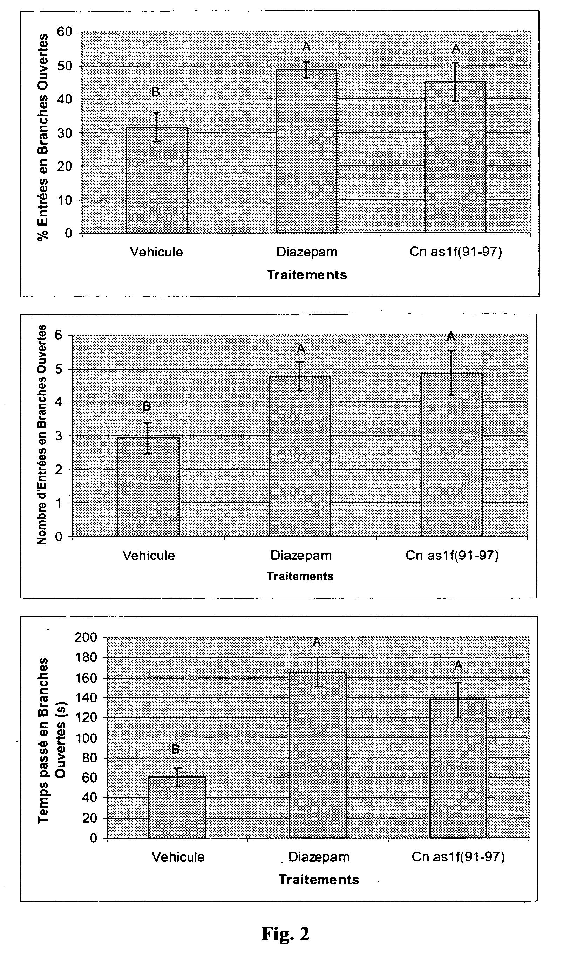

L'effet de l'injection intrapéritonéale chez le rat Wistar de 2 mL/kg d'une solution contenant 1% (v/v) de glycérol et 0,2% (v/v) de méthylcellulose (n=15) ou de 1 mg/kg de diazépam (n=15) ou de 0,7 mg/kg de l'heptapeptide CNaₛ₁-f(91-97) (n=15) sur (i) le pourcentage d'entrées dans les bras ouverts, (ii) sur le nombre d'entrées dans les bras ouverts, (iii) sur le temps passé dans les bras ouverts a été étudiée par analyse de variance non paramétrique (Kruskall-Wallis) et les comparaisons de moyennes ont été réalisées par le test de Mann-Whitney (A,B moyennes pour chaque traitement significativement différentes *p*<0,05, figure 2). Le nombre d'entrées dans les bras ouverts, le pourcentage d'entrées dans les bras ouverts, le temps passé dans les bras ouverts des animaux traités avec le véhicule sont significativement inférieurs à ceux des animaux traités par le diazépam et à celle des animaux traités par le fragment 91-97 de la caséine αₛ₁. Aucune différence significative n'est constatée entre le diazépam et le fragment 91-97 de la caséine αₛ₁. Le fragment 91-97 de la caséine αₛ₁ présente, dans le test du labyrinthe en croix surélevé chez le rat Wistar, une action similaire à celle exercée par le diazépam.

### 2.3. Boîte claire/obscure

### 2.3.1. Principe

Les tests clair/obscur sont basé sur l'aversion innée des rongeurs pour les environnements fortement éclairés et sur leur comportement exploratoire spontané en réponse à des facteurs de stress légers comme un nouvel environnement et la lumière. Le dispositif de la boîte claire/obscure permet d'étudier le comportement exploratoire du rongeur vis-à-vis d'un compartiment non familier aversif (fortement éclairé en lumière blanche) après habituation dans un compartiment non aversif (sombre) qui devient alors familier [BOURIN et HASCOËT, 2003 (18)]. Les anxiolytiques classiques peuvent être détectés en utilisant ce dispositif.

### 2.3.2. Appareillage et protocole

La boîte claire/obscure mesure 65 x 49 x 35 cm (h x Lx1) et est séparée en deux compartiments identiques par une plaque percée de trois portes de 8 x 8 cm. Le sol est recouvert de sciure. Chaque rat est placé pendant 24 h dans le compartiment arrière de la boîte, les portes de communication avec le compartiment avant étant inaccessibles. Le compartiment arrière devient ainsi le compartiment familier. L'animal est nourri et hydraté *ad libitum.* Le jour du test, le rat, après traitement, est replacé dans le compartiment familier et les portes communicant avec le compartiment avant (non familier) sont rendues accessibles afin que l'animal puisse explorer librement l'environnement. Le compartiment non familier est rendu aversif par un éclairage en lumière blanche d'une intensité de 1500 lux. Les animaux sont observés pendant 10 min et le temps passé dans chacun des compartiments est mesuré.

### 2.3.3. Traitements

L'étude a portée sur 36 rats répartis en trois groupes :
- un groupe témoin (12 rats) ayant reçu 2 mL/kg d'une solution contenant 1% (v/v) de glycérol et 0,2% (v/v) de méthylcellulose (véhicule),
- un groupe traité avec du diazépam (Valium®, Roche, Neuilly-sur-Seine, France) à la dose de 1 mg/kg mis en suspension dans le véhicule (12 rats) servant de contrôle positif,
- un groupe traité avec le fragment 91-97 de la caséine αₛ₁, préalablement dissous dans le véhicule, à la dose de 0,7 mg/kg (12 rats).

Les animaux reçoivent leur traitement respectif par voie intra-péritonéale une demi-heure avant l'ouverture de la communication entre le compartiment familier et le compartiment non-familier.

### 2.3.4. Résultats

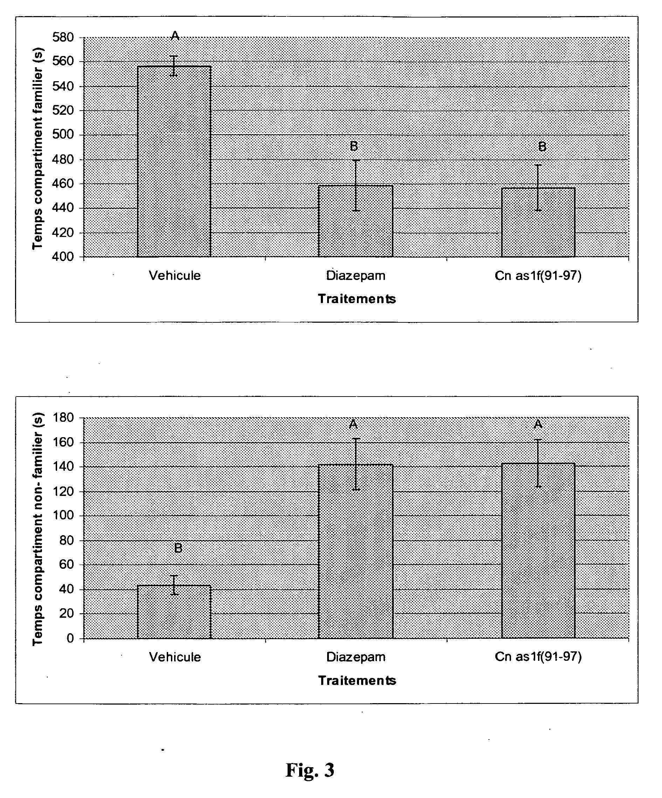

L'effet de l'injection intrapéritonéale chez le rat Wistar de 2 mL/kg d'une solution contenant 1% (v/v) de glycérol et 0,2% (v/v) de méthylcellulose (n=12) ou de 1 mg/kg de diazépam (n=12) ou de 0,7 mg/kg de l'heptapeptide CNαₛ₁-f(91-97) (n=12) sur le temps passé dans le compartiment familier et sur le temps passé dans le compartiment non-familier a été étudiée par analyse de variance non paramétrique (Kruskall-Wallis) et les comparaisons de moyennes ont été réalisées par le test de Mann-Whitney (a,b moyennes pour chaque traitement significativement différentes *p*<0,05, figure 3). Le temps passé dans le compartiment non-familier aversif des animaux traités avec le véhicule sont significativement inférieurs à ceux des animaux traités par le diazépam et à celle des animaux traités par le fragment 91-97 de la caséine αₛ₁. Aucune différence significative n'est constatée entre le diazépam et le fragment 91-97 de la caséine αₛ₁. Le fragment 91-97 de la caséine αₛ₁ montre une action similaire à celle du diazépam dans le test de la boîte claire%bscure chez le rat Wistar.

### RÉFÉRENCES BIBLIOGRAPHIQUES

(1) MERCIER, J.C., GROSCLAUDE, F., and RIBADEAU-DUMAS, B., 1971, Structure primaire de la caséine αs1 bovine. Séquence complète. Eur. J. Biochem., 23, 41-51.
(2) NAGAO, M., MAKI, M., SASAKI, R., and CHIBA, H., 1984, Isolation and séquence analysis of bovine αs1-casein cDNA clone. Agric. Biol. Chem., 48, 1663-1667.
(3) ZIOUDROU, C., STREATY, R.A., and KLEE, W.A., 1979, Opioid peptides derived from food proteins: the exorphins. J. Biol. Chem., 254, 2446-2449.
(4) LOUKAS, S., VAROUCHA, D., ZIOUDROU, C., STREATY, R.A., and KLEE, W.A., 1983, Opiod activities and structure of α-casein-derived exorphins. Biochemistry, 22, 4567-4573.
(5) MARUYAMA, S., and SUZUKI, H., 1982, A peptide inhibitor of angiotensin I converting enzyme in the tryptic hydrolysate of casein. Agric. Biol. Chem., 46, 1393-1394.
(6) MARUYAMA, S., MITACHI, H., AWAYA, J., KURONO, M., TOMIZUKA, N., and SUZUKI, H., 1987, Angiotensin 1-converting enzyme inhibitory activity of the C-terminal hexapeptide of αs1-casein. Agric. Biol. Chem., 51,2557-2561.
(7) MICLO, L., PERRIN, E., DRIOU, A., BOUDIER, J.-F., IUNG, C., and LINDEN G., 1995, Utilisation d'un décapeptide à activité de type benzodiazépine pour la préparation de médicaments et de compléments alimentaires. Brevet européen n°EP 0714910A1.
(8) MICLO, L., PERRIN, E., DRIOU, A., PAPADOPOULOS, V., BOUJRAD, N., VANDERESSE, R., BOUDIER, J.-F., DESOR, D., LINDEN, G., and GAILLARD, J.-L., 2001, Characterization of α-casozepine, a tryptic peptide from bovine αsl-casein with benzodiazepine-like activity. FASEB J. (June 8, 2001) 10.1096/fj.00-0685fje.
(9) LECOUVEY, M., FROCHOT, C., MICLO, L., ORLEWSKI, P., DRIOU, A., LINDEN, A., GAILLARD, J.-L., MARRAUD, M., CUNG, M.-T. and VANDERESSE R., 1997, Two dimentional 1H-NMR and CD structural analysis in a micellar medium of a bovin αs1-casein fragment having benzodiazepine-like properties. Eur. J. Biochem., 248, 872-878.
(10) FROCHOT, C. 1998, Étude d'un décapeptide à activité de type benzodiazépine issu d'une protéine du lait bovin. Thèse de l'Institut Polytechnique de Lorraine (INPL).
(11) NITSCHMANN, H.S., and LEHMANN, W., 1947, Zum Problem der Labwirkung auf Casein, Helv. Chim. Acta, 130, 804.
(12) THOMSON, A.R, 1984, Recent developments in protein recovery and purification. J. Chem. Tech. Biotechnol., 34B, 190-198.
(13) MAUBOIS, J.L, 1984. Separation, extraction and fractionation of milk protein components. Lait, 64, 485-495.
(14) SANOGO, T., PÂQUET, D., AUBERT, F., and LINDEN, G., 1989, Purification of αs1-casein by Fast Protein Liquid Chromatography. J. Dairy Sci., 72, 2242-2246.
(15) MERRIFIELD, R.B., 1963, Solid phase peptide synthesis. I. Synthesis of a tetrapeptide. J. Am. Chem. Soc., 85, 2149-2154.
(16) PINEL, J.P.J., and TREIT, D., 1978, Burying as a defensive response in rats. J. Comp. Physiol. Psychol. 92, 708-712.
(17) PELLOW, S., CHOPIN, P., FILE, S.E., and BRILEY, M., 1985, Validation of open : closed arm entries in an elevated plus-maze as a measure of anxiety in the rat. J. Neurosci. Methods, 14, 149-167.
(18) BOURIN, M., and HASCOËT, M., 2003, The mouse light/dark box test. Eur. J. Pharmacol., 463, 55-65.

## Revendications

1. Peptide isolé **caractérisé en ce qu'**il est choisi parmi les peptides ayant la SEQ ID NO. 1 ou la SEQ ID No. -3.

2. Polynucléotide isolé **caractérisé en ce qu'**il code pour un peptide selon la revendication 1.

3. Organisme hôte, à l'exclusion de l'homme, **caractérisé en ce qu'**il exprime un peptide selon la revendication 1.

4. Hydrolysat enzymatique de la caséine **caractérisé en ce qu'**il comprend un peptide selon la revendication 1.

5. Hydrolysat enzymatique de la caséine comprenant un peptide choisi parmi les peptides ayant les SEQ ID Nos. 1-3 pour utilisation en thérapie.

6. Peptide choisi parmi les peptides ayant les SEQ ID NOs 1.3 pour utilisation en thérapie.

7. Composition pharmaceutique **caractérisée en ce qu'**elle contient à titre de principe actif une quantité efficace d'un peptide choisi parmi les peptides ayant les SEQ ID NOs. 1-3 et/ou une quantité efficace d'un hydrolysat enzymatique de la caséine comprenant un peptide choisi parmi les peptides ayant les SEQ ID Nos. 1-3 en combinaison avec un véhicule pharmaceutique approprié.

8. Utilisation d'un peptide choisi parmi les peptides ayant les SEQ ID Nos. 1-3 et/ou d'un hydrolysat enzymatique de la caséine comprenant un peptide choisi parmi les peptides ayant les SEQ ID Nos. 1-3 pour l'obtention d'un médicament pour le traitement de l'anxiété, des troubles du sommeil et de l'épilepsie.

9. Peptide choisi parmi les peptides ayant les SEQ ID Nos. 1-3 ou hydrolysat enzymatique de la caséine comprenant un peptide choisi parmi les peptides ayant les SEQ ID Nos. 1-3 pour utilisation dans le traitement de l'anxiété, des troubles du sommeil et de l'épilepsie.

10. Composition alimentaire **caractérisée en ce qu'**elle contient une quantité efficace d'un peptide selon la revendication 1 et/ou une quantité efficace d'un hydrolysat selon la revendication 4.

11. Complément alimentaire **caractérisé en ce qu'**il contient une quantité efficace d'un peptide selon la revendication 1 et/ou une quantité efficace d'un hydrolysat selon la revendication 4 en combinaison avec des supports alimentaires de nature protéique ou glucidique.

12. Composition alimentaire ou complément alimentaire **caractérisé en ce qu'**il contient un peptide choisi parmi les peptides ayant les SEQ ID Nos. 1-3 et/ou un d'un hydrolysat enzymatique de la caséine comprenant un peptide choisi parmi les peptides ayant les SEQ ID Nos. 1-3 pour utilisation pour le traitement de l'anxiété, des troubles du sommeil et de l'épilepsie.

13. Procédé de préparation de peptides ayant une activité anxiolytique **caractérisé en ce qu'**il comprend les étapes suivantes :
- hydrolyse enzyimatique de la caséine ;
- isolement d'au moins un peptide choisi parmi les peptides ayant les SEQ ID NO*.* 1 ou la SEQ ID NO. -3.

## Claims

1. An isolated peptide **characterized in that** it is selected from peptides having SEQ ID NO. 1 or SEQ ID NO. 3.

2. An isolated polynucleotide **characterized in that** it codes for a peptide of claim 1.

3. A host organism, excluding man, **characterized in that** it expresses a peptide of claim 1.

4. A casein enzymatic hydrolysate **characterized in that** it comprises a peptide of claim 1.

5. A casein enzymatic hydrolysate comprising a peptide selected from peptides having SEQ ID NOs. 1-3 for use in therapy.

6. A peptide selected from the peptides having SEQ ID NOs. 1-3 for use in therapy.

7. A pharmaceutical composition **characterized in that** it contains as active principle an effective quantity of a peptide selected from peptides having SEQ ID NOs. 1-3 and/or an effective quantity of casein enzymatic hydrolysate comprising a peptide selected from peptides having SEQ ID NOs. 1-3 in combination with a pharmaceutically suitable carrier.

8. Use of a peptide selected from peptides having SEQ ID NOs. 1-3 and/or a casein enzymatic hydrolysate comprising a peptide selected from peptides having SEQ ID NOs. 1-3 to obtain a drug for the treatment of anxiety, sleep disorders and epilepsy.

9. A peptide selected from peptides having SEQ ID NOs. 1-3 or a casein enzymatic hydrolysate comprising a peptide selected from peptides having SEQ ID NOs. 1-3 for use in the treatment of anxiety, sleep disorders and epilepsy.

10. A dietary composition **characterized in that** it contains an effective quantity of a peptide of claim 1 and/or an effective quantity of a hydrolysate of claim 4.

11. A dietary supplement **characterized in that** it contains an effective quantity of a peptide of claim 1 and/or an effective quantity of a hydrolysate of claim 4 in combination with dietary supports containing proteins or carbohydrates.

12. A dietary composition or dietary supplement **characterized in that** it contains a peptide selected from peptides having SEQ ID NOs. 1-3 and/or a casein enzymatic hydrolysate comprising a peptide selected from peptides having SEQ ID NOs. 1-3 for use in the treatment of anxiety, sleep disorders and epilepsy.

13. A method for preparing peptides having anxiolytic activity **characterized in that** it comprises the following steps:
- enzymatic hydrolysis of casein;
- isolation of at least one peptide selected from the peptides having SEQ ID NOs. 1 or SEQ ID NO. 3.

## Patentansprüche

1. Isoliertes Peptid, **dadurch gekennzeichnet, dass** es ausgewählt ist aus den Peptiden, die die SEQ ID NO:1 oder die SEQ ID NO:3 aufweisen.

2. Isoliertes Polynucleotid, **dadurch gekennzeichnet, dass** es ein Peptid nach Anspruch 1 codiert.

3. Wirtsorganismus, mit Ausnahme des Menschen, **dadurch gekennzeichnet, dass** er ein Peptid nach Anspruch 1 exprimiert.

4. Enzymatisches Caseinhydrolysat, **dadurch gekennzeichnet, dass** es ein Peptid nach Anspruch 1 umfasst.

5. Enzymatisches Caseinhydrolysat, umfassend ein Peptid, das ausgewählt ist aus den Peptiden, die die SEQ ID NOs:1-3 aufweisen, zur Verwendung in der Therapie.

6. Peptid, ausgewählt aus den Peptiden, die die SEQ ID NOs:1-3 aufweisen, zur Verwendung in der Therapie.

7. Arzneimittel, **dadurch gekennzeichnet, dass** es als Wirkstoff eine wirksame Menge eines Peptids enthält, das ausgewählt ist aus den Peptiden, die die SEQ ID NOs:1-3 aufweisen, und/oder eine wirksame Menge eines enzymatischen Caseinhydrolysats, das ein Peptid umfasst, das ausgewählt ist aus den Peptiden, die die SEQ ID NOs:1-3 aufweisen, in Kombination mit einem verträglichen pharmazeutischen Träger.

8. Verwendung eines Peptids, ausgewählt aus den Peptiden, die die SEQ ID NOs:1-3 aufweisen, und/oder eines enzymatischen Caseinhydrolysats, das ein Peptid umfasst, das ausgewählt ist aus den Peptiden, die die SEQ ID NOs:1-3 aufweisen, zum Erhalt eines Medikaments zur Behandlung von Angst, Schlafstörungen und Epilepsie.

9. Peptid, ausgewählt aus den Peptiden, die die SEQ ID NOs:1-3 aufweisen, oder enzymatisches Caseinhydrolysat enthaltend ein Peptid, das ausgewählt ist aus den Peptiden, die die SEQ ID NOs:1-3 aufweisen, zur Verwendung in der Behandlung von Angst, Schlafstörungen und Epilepsie.

10. Nahrungsmittel, **dadurch gekennzeichnet, dass** es eine wirksame Menge eines Peptids nach Anspruch 1 und/oder eine wirksame Menge eines Hydrolysats nach Anspruch 4 enthält.

11. Nahrungsergänzungsmittel, **dadurch gekennzeichnet**, das es eine wirksame Menge eines Peptids nach Anspruch 1 und/oder eine wirksame Menge eines Hydrolysats nach Anspruch 4 in Kombination mit proteinhaltigen oder kohlenhydrathaltigen nahrungsmitteltauglichen Trägern enthält.

12. Nahrungsmittel oder Nahrungsergänzungsmittel, **dadurch gekennzeichnet, dass** es ein Peptid enthält, das ausgewählt ist aus den Peptiden, die die SEQ ID NOs:1-3 aufweisen, und/oder ein enzymatisches Caseinhydrolysat, das ein Peptid umfasst, das ausgewählt ist aus den Peptiden, die die SEQ ID NOs:1-3 aufweisen, zur Verwendung für die Behandlung von Angst, Schlafstörungen und Epilepsie.

13. Verfahren zur Herstellung von Peptiden, die eine anxiolytische Aktivität haben, **dadurch gekennzeichnet, dass** es die folgenden Schritte umfasst:
- enzymatische Hydrolyse von Casein
- Isolieren mindestens eines Peptids, ausgewählt aus den Peptiden, die die SEQ ID NO:1 oder die SEQ ID NO:3 aufweisen.
